# EUROPÄISCHE PATENTSCHRIFT

(11) **EP 2 833 120 B1**
(45) Veröffentlichungstag und Bekanntmachung des Hinweises auf die Patenterteilung: **28.09.2016**
(21) Anmeldenummer: 13003762.5
(22) Anmeldetag: 29.07.2013
(51) Int. Cl.: G01N 3/58

(54) **Kalibrierung für Bohrwiderstandsmessgeräte**
Calibration for drill resistance measuring devices
Étalonnage pour dispositifs de mesure de la résistance de perçage

(43) Veröffentlichungstag der Anmeldung: 04.02.2015
(73) Patentinhaber: IML Instrumenta Mechanik Labor System GmbH, 69168 Wiesloch (DE)
(72) Erfinder: Hunger, Sebastian, 69168 Wiesloch (DE); Hunger, Fabian, 69181 Leimen (DE); Hunger, Erich, 76131 Karlsruhe (DE)
(74) Vertreter: Straub, Bernd

(56) Entgegenhaltungen:
- DE-A1-102005 013 752

## Beschreibung

Die Erfindung betrifft einen Kalibrieradapter für Bohrwiderstandsmessgeräte, eine Kalibriervorrichtung und eine kalibrierbare Bohrwiderstandsmessvorrichtung sowie ein Verfahren zur kalibirierten Bohrwiderstandsmessung.

Bohrwiderstandsmessungen zur Analyse von Materialien sind bekannt und werden häufig zur Überprüfung des inneren Zustands von Holzobjekten wie Bäumen, Masten, Bauholz etc. eingesetzt, können aber auch bei anderen Materialien wie beispielsweise Sandstein oder Beton eingesetzt werden. Der Bohrwiderstand, den das Messobjekt einer eindringenden Bohrnadel entgegensetzt, korreliert naturgemäß mit der Dichte des Messobjekts. Üblicherweise wird bei Bohrwiderstandsmessungen die Bohrnadel mit gleichmäßigem Vorschub in das Messobjekt gedrückt und während des Bohrvorgangs die dafür erforderliche Energie in Abhängigkeit zur Eindringtiefe der Nadel gemessen..Das Messergebnis stellt sich als Bohrkurve dar, in der die Amplituden Rückschlüsse auf die Bohrwiderstände zulassen und aus der Abweichungen der Eigenschaften sowie Dichteunterschiede erkannt werden können.

Um aussagekräftige und reproduzierbare Messergebnisse zu erhalten, ist eine Kalibrierung des Bohrwiderstandsmessgeräts erforderlich, um andere Faktoren, die Einfluss auf die Messbohrung haben, etwa der Zustand der verwendeten Bohrnadel, zu eliminieren.

Hersteller von Bohrwiderstandsmessgeräten bieten eine Kalibrierung als Service etwa im Rahmen jährlicher Inspektionen an, um dem Kunden auch nach längerem Einsatz der Bohrwiderstandsmessgeräte eine hohe Messgenaulgkeit und Zuverlässigkeit zu bieten.

In DE102005013752 A1 wird ein Bohrwiderstandsmessgerät beschrieben, das einen vor der Bohrnadelaustrittsöffnung angebrachten Bohrschnabel aufweist, in den zur Kalibrierung des Gerätes ein Prüfkörper eingesetzt wird.

In DE 92 17 170 U1 wird für die Dichtemessung mittels Bohrwiderstandsmessung eine Kalibrierung durch Vergleich mit gravimetrisch ermittelter Dichte eines Bohrkerns vorgenommen, Nichtinvasive Verfahren sind aber zu bevorzugen, da sie weniger schädigend sind.

In EP 2 028 473 A1 wird ein Verfahren zur Untersuchung und Beurteilung der Standfestigkeit von Holzmasten, insbesondere von Telefon- oder Strommasten offenbart, bei dem Messkennlinien durch Bohrwiderstandsmessungen am Holzmast ermittelt werden, die dem tatsächlichen Bohrwiderstand oder einer daraus abgeleiteten Größe für die Holzfestigkeit oder die Holzdichte entsprechen, Referenzkennlinien des Bohrwiderstands oder einer daraus abgeleiteten Größe der unteren Grenze für die Belastbarkeit und Schadstellen, Schadensbilder und Schadensklassen aus dem Vergleich der Messkennlinien der Bohrwiderstandsmessungen und der Referenzkennlinien ermittelt werden. Dabei erfolgt eine Kalibrierung der Bohrwiderstandswerte durch Ermittlung eines Skalierungswertes aus Mastumbruchsmessungen, der der Steigung einer Geraden durch eine Wolke von Messpunkten in einem Diagramm entspricht, in welchem gemessene Biegespannungswerte gegen berechnete Biegespannungswerte aufgetragen sind.

Aus DE 10 2009 013 069 A1 ist ein Verfahren zur Untersuchung der Beschaffenheit von säulenförmigen oder zylindrischen Abschnitten von Objekten bekannt, bei dem eine Bohrwiderstandsmessung mit der Aufnahme von Drucksignalen am Umfang des Objektabschnitts kombiniert wird, indem die Bohrnadel diskontinuierlich eingeführt wird, so dass ein Impulstomogramm erhalten wird. Vor der Durchführung des Verfahrens an einem Messobjekt kann das Verfahren an einem Kalibrierkörper bekannter Materialqualität ausgeführt werden, um Vergleichsmesswerte zu erhalten. Wird dann das Verfahren an einem Messobjekt durchgeführt, können das erhaltene Impulstomogramm und die erhaltenen Bohrwiderstandsmessungen mit den entsprechenden Vergleichsmesswerten des Kalibrierkörpers verglichen werden, so dass in Abhängigkeit der Abweichungen der erhaltenen Werte eine Schädigung des Messobjekts ermittelt werden kann.

Ausgehend von diesem Stand der Technik ist es Aufgabe der vorliegenden Erfindung, eine Vorrichtung zu schaffen, mit der flexibel vielerorts die Kalibrierung einer mit einem Bohrwiderstandsmessgerät durchgeführten Messung in einfacher Weise ermöglicht werden kann.

Diese Aufgabe wird durch einen Kalibrieradapter mit den Merkmalen des Anspruchs 1 gelöst.

Eine entsprechende Kalibriervorrichtung, mit der Bohrwiderstandsmessgeräte kalibriert werden können, wird mit den Merkmalen des Anspruchs 5 offenbart

Die Aufgabe, eine Bohrwiderstandsmessvorrichtung bereitzustellen, mit der ohne aufwändige Auswertungen und Vergleiche kalibrierte Bohrwiderstandsmessungen in einfacher Weise durchgeführt werden können, wird durch die Bohrwiderstandsmessvorrichtung mit den Merkmalen des Anspruchs 9 gelöst.

Eine kalibrierte Bohrwiderstandsmessung lässt sich durch ein Verfahren mit den Merkmalen des Anspruchs 10 ausführen.

Weiterbildungen der Gegenstände und des Verfahrens sind in den jeweiligen Unteransprüchen ausgeführt.

Ein erfindungsgemäßer Kalibrieradapter ist zum Aufsetzen auf ein Bohrwiderstandsmessgerät ausgebildet, das üblicherweise zumindest eine mit einem Antrieb gekoppelte Bohrnadelaufnahme und eine durch eine Austrittsöffnung in einer Führungshülse geführte Bohrnadel, sowie eine Erfassungs- und Ausgabevorrichtung des gemessenen Bohrwiderstands aufweist. Der Bohrwiderstand wird beim Eindringen in das zu untersuchende Material erfahren.

Der Kalibrieradapter weist einen Grundkörper mit einer Kopplungsvorrichtung auf, durch die der Kalibrieradapter lösbar an dem Bohrwiderstandsmessgerät vor der Bohrnadelaustrittsöffnung befestigt werden kann. Für unterschiedlich gestaltete Bohrwiderstandsmessgeräte können entsprechend unterschiedliche, an das jeweilige Bohrwiderstandsmessgerät angepasste Kopplungsvorrichtungen vorgesehen sein. Durch den Grundkörper des Kalibrieradapters erstreckt sich ein Durchtrittskanal für die Bohrnadel, der so ausgebildet ist, dass er bei Befestigung des Kalibrieradapters an dem Bohrwiderstandsmessgerät mit der Bohrnadelaustrittsöffnung fluchtet. Zudem weist der Kalibrieradapter in dem Grundkörper einen Aufnahmeraum auf, in dem ein Prüfkörper zumindest teilweise aufgenommen werden kann. Eine durch den Durchtrittskanal definierte Fluchtachse schneidet den Aufnahmeraum so, dass der in dem Aufnahmeraum aufgenommene Prüfkörper mit seinem zu prüfenden Abschnitt (Prüfabschnitt) vor dem Durchtrittskanal zu liegen kommt, um dort von der Bohrnadel des Bohrwiderstandsmessgeräts durchbohrt zu werden.

Der erfindungsgemäße Kalibrieradapter kann auch zwei oder mehr Aufnahmeräume für zwei oder mehr Prüfkörper aufweisen, bzw. ein einziger Aufnahmeraum kann so ausgebildet sein, dass zwei oder mehr Prüfkörper aufgenommen werden können. Da jeder Aufnahmeraum in der Fluchtachse des Durchtrittskanals liegt, kommen die Aufnahmeräume in dem Grundkörper in Bezug zu der Fluchtachse hintereinander zu liegen, wobei die Aufnahmeräume durch Wandungen voneinander getrennt sein können oder ineinander übergehen können.

Natürlich kann der Kalibrieradapter für mehrere Bohrwiderstandsmessgeräte nacheinander oder für unterschiedliche Typen eingesetzt werden.

Vorteilhaft können mit dem Kalibrieradapter flexibel nahezu jederzeit und fast an jedem Ort kalibrierte Messvorgänge mit dem Bohrwiderstandsmessgerät durchgeführt werden. Einflüsse auf das Messergebnis wie etwa der Zustand der Bohrnadel können durch Nutzung des Kalibrieradapters eliminiert werden, Mittels des Kalibrieradapters kann ein vorgegebener Prüfkörper definiert vor der Bohrnadel platziert und direkt vor der eigentlichen Messbohrung durchdrungen werden, wobei exaktere und reproduzierbare Ergebnisse erhalten werden als ohne Kalibrierung.

Generell ist denkbar, dass der Grundkörper den Prüfkörper nicht vollständig umschließt, so dass eine von dem Durchtrittskanal abgewandte Seite des Prüfkörpers freiliegt. Der Prüfkörper könnte durch einen Umgriff oder eine Klammer vor dem Durchtrittskanal gehalten werden, so dass die Bohrnadel nach Durchdringen des Prüfkörpers direkt austritt und in ein Messobjekt eingeführt werden kann. Bevorzugt jedoch wird der Prüfköper in Bohrnadelvorschubrichtung beidseitig durch den Grundkörper eingefasst sein, so dass der Durchtrittskanal beidseitig des Aufnahmeraums durch den Grundkörper verläuft. So könnte der Aufnahmeraum einfach als eine Art Einschubfach in dem Grundkörper konzipiert sein, in den der Prüfkörper eingeschoben werden kann. Damit ein Prüfkörper für mehrere Kalibrierungsbohrungen genutzt werden kann, ist der Prüfkörper in einer Ebene senkrecht zu der Fluchtachse, die durch den Durchtrittskanal definiert wird, in dem Aufnahmeraum auf einem oder mehreren Lagerelementen dreh- und/oder verschiebbar gelagert, so dass nach einer erfolgten Durchbohrung des Prüfkörpers an einem ersten Prüfabschnitt der Prüfkörper verschoben oder gedreht wird, so dass ein noch nicht durchbohrter Prüfabschnitt in der Fluchtachse zu liegen kommt.

Damit der Prüfkörper nicht nur in einfacher Weise eingesetzt und ausgetauscht werden kann, sondern auch vor unbeabsichtigtem Entfernen aus dem Aufnahmeraum gesichert ist, kann der Kalibrieradapter einen abnehmbaren Deckel aufweisen, der den Grundkörper ergänzt und der den Aufnahmeraum zusammen mit dem Grundkörper begrenzt, so dass der Aufnahmeraum bei Abnahme des Deckels geöffnet wird. Die im Deckel vorliegende Durchtrittsöffnung fluchtet entsprechend mit dem für die Bohrnadel vorgesehenen Durchtrittskanal im Grundkörper, so dass die Durchtrittsöffnung und der Durchtrittskanal den gesamten Durchgang für die Bohrnadel in dem Kalibrieradapter bilden.

Damit sich der dreh- und/oder verschiebbar gelagerte Prüfkörper während der Messung nicht ungewollt bewegt, kann der Kalibrieradapter eine Fixiervorrichtung haben, mit der der Prüfkörper in einer Prüfstellung fixiert werden kann. Alternativ oder zusätzlich kann der Kalibrieradapter eine Stellvorrichtung, eine Erfassungsvorrichtung und/oder eine Anzeigevorrichtung zum manuellen und/oder automatischen Positionieren des Prüfkörpers in dem Aufnahmeraum aufweisen. Eine Stellvorrichtung kann beispielsweise einen elektrischen Antrieb umfassen, so dass der Prüfkörper nicht manuell, sondern automatisch nach jeder Messung oder nach Benutzereingabe, beispielsweise per Knopfdruck, in eine neue Prüfposition gebracht wird; eine Stellvorrichtung kann aber auch ein lediglich mechanisches Bauteil zum Bewegen eines von außerhalb des Grundkörpers nicht direkt zugänglichen Prüfkörpers sein. Mit einer Erfassungsvorrichtung kann eine Lage des Prüfkörpers und/oder die Anzahl der an dem Prüfkörper vorgenommenen Bohrungen erfasst werden. Eine solche Erfassungsvorrichtung kann sinnvoller Weise mit einer Anzeigevorrichtung gekoppelt sein, um den Nutzer wissen zu lassen, wann ein Prüfkörper ausgetauscht werden muss. Eine Anzeigevorrichtung kann aber auch lediglich durch Markierungen an dem Rand einer Prüfscheibe gegeben sein, die Umfangabschnitte zum manuellen Überführen der Scheibe in eine nächste Prüfposition markieren.

Die Kopplungsvorrichtung, mit denen der Kalibrieradapter an dem Bohrwiderstandsmessgerät lösbar befestigt wird, kann Rast- oder Klemmvorrichtungen, darunter auch Schrauben, bevorzugt Rändelschrauben, aufweisen, mit denen der Kalibrieradapter an der Bohrnadelführungshülse oder einer die Bohrnadelführungshülse einfassenden Kappe festgeklemmt werden kann. Alternativ sind als Kopplungsvorrichtung auch Stifte und Schrauben zur Befestigung des Kalibrieradapters an einer Frontplatte des Bohrwiderstandsmessgeräts vorstellbar. Weist das Bohrwiderstandsmessgerät ein entsprechendes Gegenstück auf, kommen auch Verbindungstechniken wie ein Schraub- oder Bajonettverschluss in Frage. Hat etwa die Bohrnadelführungshülse bzw. die Kappe ein Außengewinde, kann dies mit einem am Grundkörper vorliegenden Innengewinde in Eingriff gebracht werden.

Ein weiterer Gegenstand der Erfindung ist eine Kalibriervorrichtung für Bohrwiderstandsmessgeräte, die außer einem erfindungsgemäßen Kalibrieradapter zumindest einen, ebenfalls erfindungsgemäßen, Prüfkörper aufweist, der Abmessungen und eine Form aufweist, die eine Anordnung des Prüfkörpers in dem Aufnahmeraum des Kalibrieradapters gestatten. Damit ist ein Prüfabschnitt des Prüfkörpers vollständig in der durch den Durchtrittskanal definierten Fluchtachse positionierbar. Es hat sich gezeigt, dass eine durchzubohrende Mindeststärke des Prüfkörpers, um ein aussagekräftiges und reproduzierbares Signal zu ergeben, zumindest 3 mm, vorzugsweise 5 mm beträgt. Abhängig auch von der Dichte des Prüfkörpers können Prüfkörper auch andere Stärken, etwa 8 mm oder mehr, aufweisen.

Um das Bohrwiderstandsmessgerät für Messobjekte mit unterschiedlicher Dichte bzw. Festigkeit exakt kalibrieren zu können, kann die Kalibriervorrichtung zwei oder mehr Prüfkörper unterschiedlicher Werkstoffe mit bekannten, mit dem Bohrwiderstand korrelierten Werkstoffgrößen wie Dichte bzw. Festigkeit etc. umfassen, von denen jeweils einer in dem Aufnahmeraum angeordnet werden kann.

Sind mehr als ein Aufnahmeraum für Prüfkörper vorgesehen, bzw. ist der Aufnahmeraum zur Aufnahme mehrerer Prüfkörper ausgebildet, kann in jedem Aufnahmeraum zumindest ein Prüfkörper angeordnet sein. Zur Kalibrierung können die Prüfkörper aus den unterschiedlichen Werkstoffen durchbohrt werden, es kann aber durch entsprechende Anordnung der Prüfkörper in den Aufnahmeräumen auch nur jeweils ein Prüfkörper zur Kalibrierung durchbohrt werden. Die Positionierung der Prüfkörper in den Aufnahmeräumen kann händisch oder durch die Stellvorrichtung automatisiert erfolgen.

In einer bevorzugten Ausführungsform ist der Prüfkörper der Kalibriervorrichtung als runde Prüfscheibe mit einer zentrischen Bohrung ausgeprägt, für die in dem entsprechend rund ausgeformten Aufnahmeraum benachbart zu dem Durchtrittskanal ein parallel dazu angeordneter Lagerstift vorgesehen ist, so dass die Prüfscheibe drehbar im Aufnahmeraum angeordnet ist. Mit einem vorzugsweise zentriert durch den Grundkörper verlaufenden Durchtrittskanal ist die Prüfscheibe damit exzentrisch im Grundkörper gelagert, so dass dabei gleichzeitig vorteilhaft die in dem Aufnahmeraum aufgenommene Prüfscheibe teilweise aus dem Grundkörper ragt. Auf diese Weise kann die Prüfscheibe bequem und einfach mit der Hand verdreht werden, um einen nicht durchbohrten Prüfabschnitt vor dem Durchtrittskanal zu platzieren. Für eine gute Griffigkeit kann der Rand der Prüfscheibe profiliert sein.

Alternativ zur Prüfscheibe kann der Prüfkörper auch in Form einer leistenartigen Prüfplatte vorliegen, und der Aufnahmeraum erstreckt sich senkrecht zu der Fluchtachse gerade durch den Grundkörper hindurch, so dass die Prüfplatte durch den Aufnahmeraum durchgeschoben werden kann, um jeweilige Prüfabschnitte vor dem Durchtrittskanal zu platzieren.

Bei Kalibrieradaptern mit mehreren Aufnahmeräumen können auch beide Prüfkörpervarianten kombiniert werden.

Eine erfindungsgemäße Bohrwiderstandsmessvorrichtung umfasst ein Bohrwiderstandsmessgerät, das zumindest eine mit einem Antrieb gekoppelte Bohrnadelaufnahme, eine Bohrnadel, eine Führungshülse mit einer Bohrnadelaustrittsöffnung und eine Bohrwiderstanderfassung und -ausgabe hat. Ferner umfasst diese Bohrwiderstandsmessvorrichtung die erfindungsgemäße Kalibriervorrichtung, die wiederum aus dem erfindungsgemäßen Kalibrieradapter und einem wie oben beschriebenen geeigneten Prüfkörper besteht. Der Kalibrieradapter ist mittels der Kopplungsvorrichtung des Grundkörpers lösbar an dem Bohrwiderstandsmessgerät vor der Bohrnadelaustrittsöffnung befestigt. Mit dieser Bohrwiderstandsmessvorrichtung können einfach und unaufwändig kalibrierte Bohrwiderstandsmessungen durchgeführt werden.

Zur Durchführung eines erfindungsgemäßen Verfahrens zur kalibrierten Bohrwiderstandsmessung wird zunächst ein erfindungsgemäßer Kalibrieradapter herangenommen, in dessen Aufnahmeraum ein Prüfkörper aus einem vorgegebenen Werkstoff mit einer vorgegebenen, mit dem Bohrwiderstand korrelierten Werkstoffgröße wie etwa der Dichte, Festigkeit etc. platziert wird. Der Kalibrieradapter wird auf das Bohrwiderstandsmessgerät aufgesetzt und daran befestigt, wobei der Durchtrittskanal des Kalibrieradapters mit der Bohrnadelaustrittsöffnung in der Bohrnadelführungshülse fluchtet. Zur Durchführung der Bohrwiderstandsmessung mit dem Bohrwiderstandsmessgerät an einem Messobjekt wird die Bohrnadel mit gleichmäßigem Vorschub angetrieben, so dass sie zunächst den in dem Kalibrieradapter vorliegenden Prüfkörper durchdringt, wobei das Bohrwiderstandsmessgerät ein Bohrwiderstand-Kalibriersignal erfasst. Darauf folgend wird die Bohrnadel weiter vorgeschoben und in das Messobjekt eingeführt, wobei ein Bohrwiderstand-Messsignal erfasst wird. Basierend auf der Differenz zwischen dem Kalibriersignal und dem Messsignal und der vorgegebenen, mit dem Bohrwiderstand korrelierten Werkstoffgröße des Prüfkörpers kann die entsprechende Werkstoffgröße des Messobjekts berechnet werden.

Ist das Bohrwiderstandsmessgerät auf diese Weise kalibriert, werden nach Zurückziehen der Bohrnadel weitere Bohrwiderstandsmessungen am gleichen Messobjekt ohne neue Kalibrierung durchgeführt. Zur Durchführung einer weiteren kalibrierten Bohrwiderstandsmessung wird nach Zurückziehen der Bohrnadel die Lage des Prüfkörpers in dem Aufnahmeraum in einer Ebene senkrecht zur Fluchtachse verändert, so dass ein nicht durchbohrter Prüfabschnitt des Prüfkörpers vor dem Durchtrittskanal zu liegen kommt. Alternativ oder wenn sämtliche Prüfabschnitte durchbohrt sind, kann der Prüfkörper ausgetauscht werden.

Beim Bereitstellen des Kalibrieradapters zur Durchführung einer kalibrierten Messung kann bevorzugt ein Prüfkörper mit einer Werkstoffgröße ausgewählt werden, deren Größenordnung im Bereich einer zu erwartenden Werkstoffgröße des Messobjekts liegt, so dass ein noch genaueres Ergebnis erhalten wird.

Diese und weitere Vorteile werden durch die nachfolgende Beschreibung unter Bezug auf die begleitenden Figuren dargelegt. Der Bezug auf die Figuren in der Beschreibung dient der Unterstützung der Beschreibung und dem erleichterten Verständnis des Gegenstands. Gegenstände oder Teile von Gegenständen, die im Wesentlichen gleich oder ähnlich sind, können mit denselben Bezugszeichen versehen sein. Die Figuren sind lediglich eine schematische Darstellung einer Ausführungsform der Erfindung, Dabei zeigen:
- **Fig. 1**: eine perspektivische Ansicht einer Ausführungsform der erfindungsgemäßen Kalibriervorrichtung aus Kalibrieradapter und Prüfscheibe,
- **Fig. 2**: eine perspektivische Ansicht eines mit einem Kalibrieradapter ausgestatteten Bohrwiderstandsmessgeräts,
- **Fig. 3**: eine Detailansicht des auf das Bohrwiderstandsmessgerät aufgesetzten Kalibrieradapters,
- **Fig. 4**: eine teilweise Explosionsansicht des auf das Bohrwiderstandsmessgerät aufgesetzten Kalibrieradapters aus Fig. 3,
- **Fig. 5**: eine Schnittansicht von unten durch den auf das Bohrwiderstandsmessgerät aufgesetzten Kalibrieradapter aus Fig. 3,
- **Fig. 6**: eine Seitenschnittansicht durch den auf das Bohrwiderstandsmessgerät aufgesetzten Kalibrieradapter aus Fig. 3,
- **Fig. 7**: eine Frontansicht auf einen Kalibrieradapter ohne Deckel mit zwei aneinander angrenzenden, gleichartigen Aufnahmeräumen,
- **Fig. 8**: eine Frontansicht auf einen Kalibrieradapter ohne Deckel mit zwei axial voneinander beabstandeten, unterschiedlichen Aufnahmeräumen,
- **Fig. 9**: eine erste mit einer erfindungsgemäßen Bohrwiderstandsmessvorrichtung erhaltene Bohrkurve,
- **Fig. 10**: eine zweite mit einer erfindungsgemäßen Bohrwiderstandsmessvorrichtung erhaltene Bohrkurve,
- **Fig. 11**: eine dritte mit einer erfindungsgemäßen Bohrwiderstandsmessvorrichtung erhaltene Bohrkurve,
- **Fig. 12**: eine weitere Bohrkurve, der in schematischer Darstellung Prüfkörper und Messobjekt zugeordnet sind.

Die Erfindung bezieht sich auf einen Kalibrieradapter, der auf ein Bohrwiderstandsmessgerät zur Durchführung kalibrierter Bohrwiderstandsmessungen aufgesetzt werden kann.

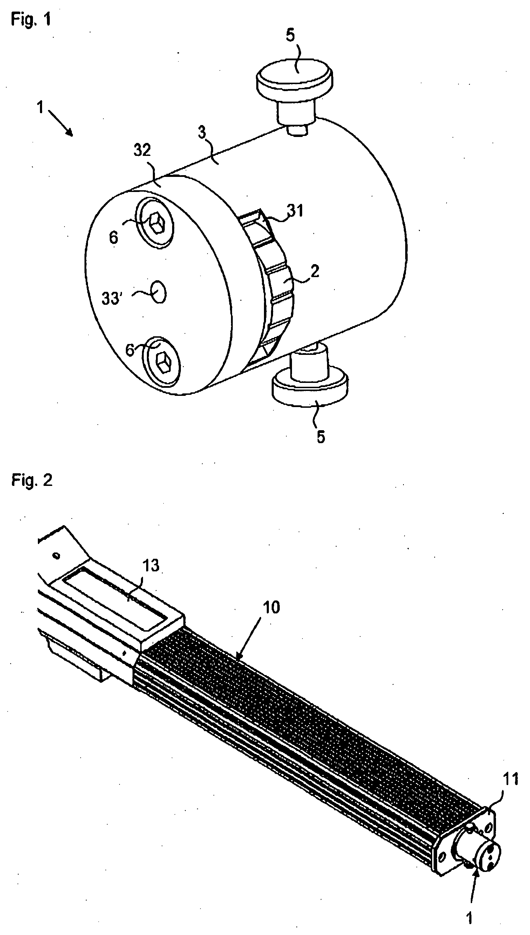

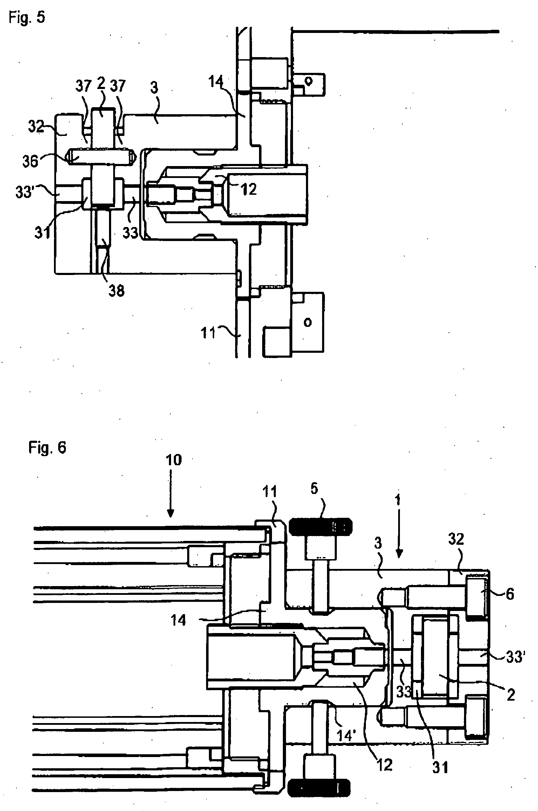

**Fig. 1** **zeigt** einen erfindungsgemäßen Kalibrieradapter 1, der auf ein Bohrwiderstandsmessgerät 10 aufgesteckt werden kann, wie in **Fig. 2** zu sehen ist, und der im vorliegenden Beispiel mit zwei Rändelschrauben 5 fixiert wird. Üblicherweise befindet sich in der Frontplatte 11 eines Bohrwiderstandsmessgeräts 10 eine Bohrnadelführungshülse 12, die im vorliegenden Fall noch von einer Kappe 14 umgeben wird (vgl. **Fig. 5** und **Fig. 6****).** Der Kalibrieradapter 1, d. h. sein Grundkörper 3, ist dabei so ausgebildet, dass er auf die Kappe 14 passgenau aufgesetzt werden kann und an der Frontplatte 11 zur Anlage kommt. Im vorliegenden Fall bedeutet dies, dass der Grundkörper 3 eine der Kappe 14 angepasste zylindrische Ausnehmung aufweist. Für andere Bohrwiderstandsmessgeräte, die nicht mit einer solchen Kappe oder auch einer anders gearteten Bohrnadelführung ausgestattet sind, können alternative Grundkörperformen oder unterschiedliche Passstücke vorgesehen sein, mit denen der Kalibrieradapter dem entsprechenden Bohrwiderstandsmessgerät angepasst werden kann.

Eine Befestigung des aufgesteckten Kalibrieradapters 1 kann, wie in den Fig. 1 bis 4 und 6 gezeigt, durch Rändelschrauben 5 ermöglicht werden, die durch Anziehen den Kalibrieradapter 1 an der Kappe 14 festklemmen.

Der passgenaue Sitz des Kalibrieradapters 1 ermöglicht, dass die aus der Bohrnadelführung 12 austretende Bohrnadel genau in den Durchtrittskanal 33 des Kalibrieradapters 1 eintritt, der zu dem Aufnahmeraum 31 mit dem Prüfkörper 2 führt, der zwischen dem Grundkörper 3 und dem Deckel 32 liegt. Der Prüfkörper 2 ist im dargestellten Beispiel eine runde Prüfscheibe 2. Diese Prüfscheibe 2 wird beim Durchführen einer Bohrwiderstandsmessung durch Vorschub der Bohrnadel durchbohrt, ehe die Bohrnadel aus der Durchtrittsöffnung 33' des Deckels 32 austritt und in das Messobjekt eindringt. Die Messsignale, die beim Durchdringen der Prüfscheibe 2 erhalten werden, dienen der Kalibrierung der direkt nachfolgenden Messung am Messobjekt.

Die Prüfscheibe 2 ist hier in Bezug zu dem Durchtrittskanal 33 exzentrisch und drehbar gelagert, so dass durch einfaches Drehen der Prüfscheibe 2 mehrere

Prüfbohrungen auf einer Prüfscheibe 2 durchgeführt werden können. Um das Drehen zu erleichtern, weist die Prüfscheibe 2 einen profilierten Rand auf. Zum einfachen Austausch der Prüfscheibe 2 ist der Deckel 32 mit zwei Schrauben 6 an dem Grundkörper 3 befestigt.

In dem erfindungsgemäßen Kalibrieradapter können verschiedene Prüfscheiben angeordnet werden, die sich hinsichtlich ihres Werkstoffes und damit hinsichtlich ihrer Dichte bzw. Festigkeit unterscheiden. Dies ist insofern von Bedeutung, als dass die Kalibrierung für die nachfolgende Messung umso exakter ausfällt, wenn die Festigkeit der Prüfscheibe der zu erwartenden Festigkeit des Messobjekts ähnlich ist. So können Prüfscheiben mit hoher Festigkeit, z. B. aus Carbon (kohlefaserverstärktem Kunststoff) mit 13 MPa zur Kalibrierung einer Messung an hartem Holz (z. B. Ahorn oder Eiche) eingesetzt werden, während Prüfscheiben aus Kunststoff, z. B. Teflon, geringere Festigkeiten aufweisen und zur Kalibrierung einer Messung an entsprechend weicherem Holz (z. B. Nadelholz) vorgesehen sein können

Weitere denkbare Werkstoffe für Prüfkörper sind jegliche Werkstoffe aus homogenem Material, dessen Eigenschaften bekannt sind, die sich zur Messung an verschiedenen Materialien wie Holz oder Stein eignen, also Materialien mit eventuell inhomogener Dichte, die zu untersuchen ist. Der Prüfkörper weist sinnvoller Weise eine homogene Dichte und Festigkeit auf, so dass ein klares, reproduzierbares Kalibriersignal erhalten wird.

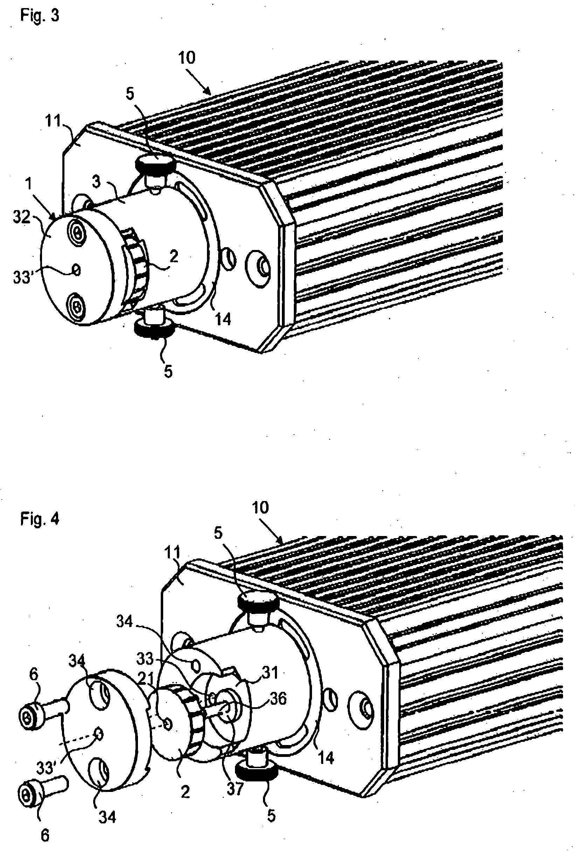

**Fig. 3** zeigt den auf das Bohrwiderstandsmessgerät 10 aufgesetzten Kalibrieradapter 1, der in **Fig. 4** in einer teilweisen Explosionsansicht zu sehen ist, In der der Deckel 32 und die Prüfscheibe 2 von dem Grundkörper 3 abgehoben sind. Zur passgenauen Anordnung des Deckels 32 an dem Grundkörper 3, damit der Durchtrittskanal 33 und die Durchtrittsöffnung 33' für die Bohrnadel exakt fluchten, sind im Grundkörper 3 und im Deckel 32 ebenfalls fluchtende Bohrungen 34 zur Aufnahme der Schrauben 6 vorgesehen. In der Explosionsdarstellung ist der zu dem Durchtrittskanal 33 versetzte Lagerstift 36 zu sehen, auf dem die Prüfscheibe 2 mit ihrer zentrischen Bohrung 21 angeordnet wird. Ein im Grundkörper 3 und im Deckel 32 (siehe **Fig. 5****)** vorliegender Absatz 37 sorgt für eine axiale Fixierung der Prüfscheibe 2 **im** Aufnahmeraum 31.

In den Schnittansichten von unten **(****Fig. 5****) und** von der Seite **(****Fig. 6****)** ist neben der Lagerung der Prüfscheibe 2 in dem Aufnahmeraum 31 zwischen Grundkörper 3 und Deckel 32 der passgenaue Sitz des Grundkörpers 3 auf der Kappe 14 des Bohrwiderstandsmessgeräts 10 zu sehen. In dem Außenumfang des Abschnitts der Kappe 14, der die Bohrnadelführungshülse 12 umschließt, ist eine Nut 14' eingebracht, in die die Rändelschrauben 5 eingreifen, wie in **Fig. 6** zu sehen ist.

In **Fig. 5** ist zudem ein in dem Grundkörper 3 vorgesehener Haltestift 38 zu sehen, dessen Kopf in die Aufnahmeöffnung 31 hineinragt und mit der Prüfscheibe 2 in Eingriff steht, um die Prüfscheibe 2 während des Messvorgangs zu fixieren, so dass diese nicht ungewollt rotiert wird. Vorteilhaft können hierzu Kerben des profilierten Rands der Prüfscheibe 2 als Eingriffspositionen genutzt werden. Der Haltestift 38 kann mit einer Vorspannung für diese Eingriffspositionen versehen sein, beispielsweise mittels einer Feder. Diese Vorspannung ist ausreichend zur Fixierung der Prüfscheibe 2 während des Messvorgangs, kann aber beim Drehen der Prüfscheibe 2 leicht überwunden werden, so dass der Haltestift 38 aus der Kerbe im Rand der Prüfscheibe 2 zurückgedrängt wird. Der Kopf des Haltestifts 38 kann somit eine Rastnase bilden, was insbesondere von Vorteil ist, wenn die umfänglichen Abstände zwischen den Kerben so gewählt sind, dass die Prüfscheibe 2, wenn sie nach erfolgter Messung durch Drehen in eine nächste Prüfposition überführt werden soll, beim Einrasten in der nächsten Kerbe automatisch die nächste Prüfposition einnimmt, In der ein nicht durchbohrter Abschnitt vor dem Durchtrittskanal 33 zu liegen kommt.

Es ist nicht zwingend erforderlich, dass vor jeder Messung eine Kalibrierung erfolgt. So kann die Prüfscheibe auch in ihrer Position belassen werden, wenn etwa mehrere Messungen an einem Messobjekt erfolgen oder wenn mehrere gleichartige Messobjekte untersucht werden sollen. Dann passiert die Bohrnadel nach erfolgter Kalibrierung in den nachfolgenden Messungen die durchbohrte Stelle in der Prüfscheibe ohne neuerliche Kalibrierung.

Alternativ oder zusätzlich zu Kerben in passendem Abstand können auch andere Markierungen auf der Prüfscheibe 2 bzw. deren Rand vorgesehen sein, die dem Bediener anzeigen, wie weit er die Prüfscheibe 2 bewegen muss, um eine nächste Prüfposition zu erreichen. Ferner kann insbesondere in Verbindung mit einem rastenden Haltestift 38 ein Zählwerk oder ähnliches integriert werden, so dass dem Bediener angezeigt werden kann, wenn alle Prüfpositionen der Prüfscheibe 2 genutzt worden sind und die Prüfscheibe 2 ausgetauscht werden muss.

**Fig. 1 bis 6** zeigen den Prüfkörper beispielhaft als eine runde Scheibe, die sich vorteilhaft zur Durchführung weiterer kalibrierter Messungen einfach drehen lässt. Es sind aber auch andere Geometrien für einen Prüfkörper denkbar, etwa rechteckige, leistenähnliche Formen, die dann beispielsweise durch den Aufnahmeraum geschoben anstatt gedreht werden können, um weitere Prüfpositionen einzunehmen.

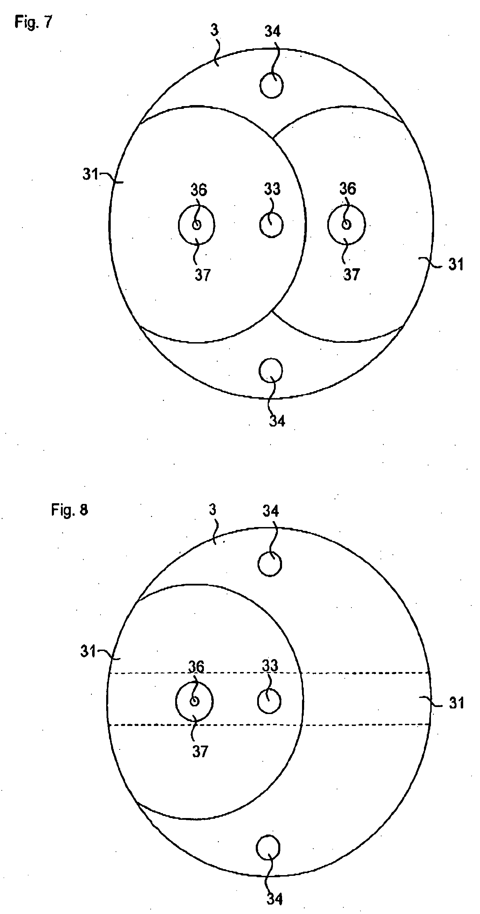

**Fig. 7** und **Fig. 8** zeigen beispielhaft zwei mögliche Varianten eines erfindungsgemäßen Kalibrieradapters, in dessen Grundkörper 3 zwei Aufnahmeräume 31 vorgesehen sind. In Fig. 7 liegen die beiden Aufnahmeräume 31 axial hintereinander versetzt; die durch den Durchtrittskanal 33 definierte Fluchtachse schneidet somit beide Aufnahmeräume 31. Der in der Zeichenebene weiter hinten liegende, linke Aufnahmeraum 31 weist wie der weiter vorne liegende einen Absatz 37 zur axialen Fixierung einer auf dem Lagerstift 36 aufgenommenen Prüfscheibe (in **Fig. 7** nicht dargestellt) auf. Das im nicht gezeigten Deckel vorliegende Gegenlager ist korrespondierend dazu ausgebildet, d. h. der dem linken "entfernteren" Aufnahmeraum 31 zugeordnete Absatz zur axialen Fixierung ragt weiter von dem Deckel hervor als der dem rechten "näheren" Aufnahmeraum 31.

**Fig. 8** skizziert einen erfindungsgemäßen Kalibrieradapter, der einen wie oben beschriebenen runden Aufnahmeraum 31, der durch den (nicht dargestellten) Deckel begrenzt wird, und der als zweiten Aufnahmeraum 31 ein sich linear durch den Grundkörper 3 erstreckendes Schubfach 31 aufweist, das in der Zeichenebene weiter hinten liegt und daher gestrichelt dargestellt ist. In diesem Schubfach kann eine entsprechende leistenartige Prüfplatte angeordnet werden.

In den Kalibrieradaptern mit mehr als einem Aufnahmeraum müssen nicht zwangsläufig mehrere Prüfkörper angeordnet sein; es kann auch nur einer der Aufnahmeräume genutzt werden. Bei Durchführung der kalibrierten Bohrwiderstandsmessungen können bei Bedarf mehrere Prüfscheiben mit unterschiedlichen Dichten durchbohrt und so mehrere Kalibriersignale erhalten werden, es ist aber auch möglich, dass nur jeweils eine Prüfscheibe, deren Dichte in etwa der zu erwartenden Dichte des Messobjekts entspricht, in Prüfposition mit einem undurchbohrten Abschnitt vor dem Durchtrittskanal gebracht wird. Der oder die anderen Prüfkörper können außerhalb der Fluchtachse angeordnet werden oder mit einem bereits durchbohrten Prüfabschnitt in der Fluchtachse positioniert werden, so dass die Bohrnadel diesen Prüfkörper nicht erneut durchbohrt.

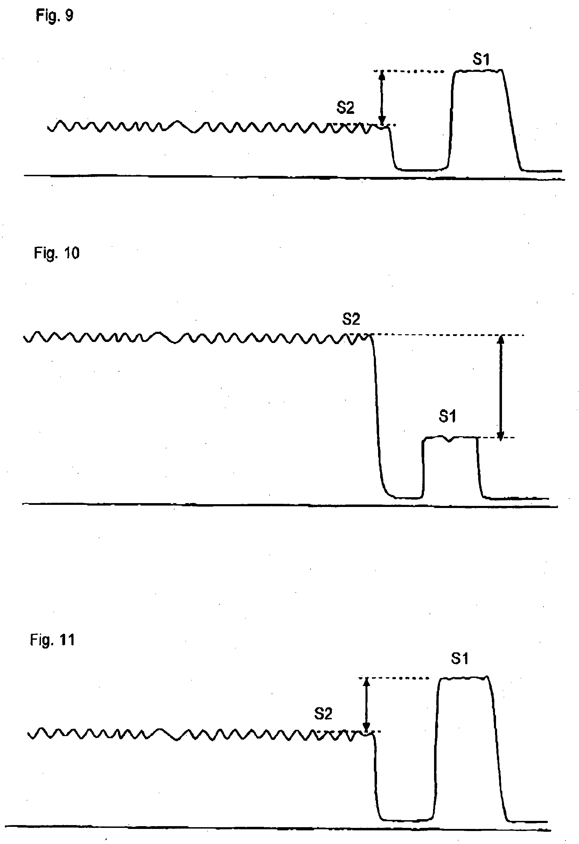

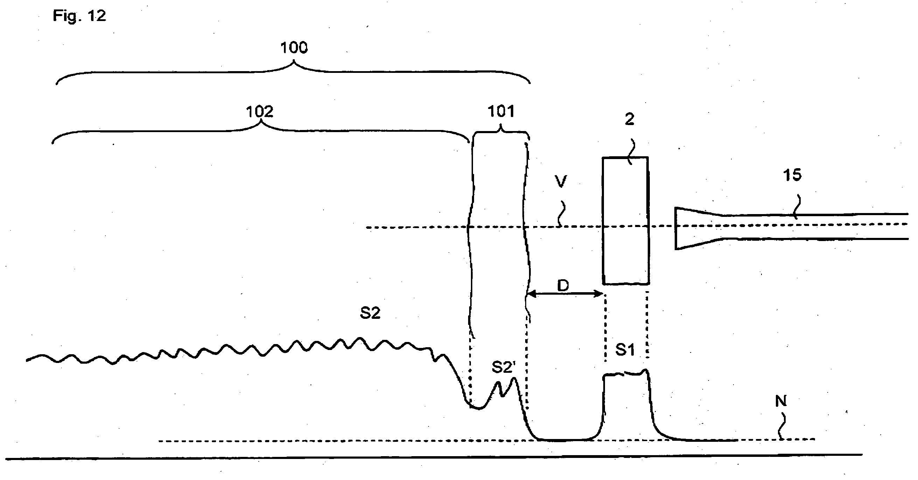

**Fig. 9** bis **11** zeigen Bohrkurven, wie sie bei erfindungsgemäß durchgeführten kalibrierten Bohrwiderstandsmessungen erhalten werden. **Fig. 12** verdeutlicht, wie die Messsignale S1 und S2 einer Bohrkurve erhalten werden. Zur Durchführung einer kalibrierten Bohrwiderständsmessung wird die Bohrnadel 15 vorgeschoben und durchdringt zunächst den mittels des (hier nicht dargestellten) Kalibrieradapters in der Vorschubrichtung V (= Fluchtachse des Durchtrittskanals) platzierten Prüfkörper 2, wodurch das Kalibriersignal S1 aufgenommen wird, ehe die Bohrnadel 15 in das Messobjekt 100, beispielsweise einen Baum, eindringt, wobei das Messsignal S2 erhalten wird. Das Messsignal S2' entsteht beim Durchdringen der Baumrinde 101 aus Borke und Bast, deren Dichte von der Dichte des Holzes 102 im Inneren des Baumes 100 abweicht. Zwischen Prüfkörper 2 und Messobjekt 100 ist von der Bohrnadel 15 eine gewisse, durch den Deckel des Kalibrieradapters vorbestimmte Distanz D zurückzulegen, in der die Bohrnadel 15 durch die Durchtrittsöffnung in dem Deckel tritt und keinen Bohrwiderstand erfährt, wie sich in der Bohrkurve durch den Abfall auf die Nulllinie N zeigt.

Generell wird zur erfindungsgemäßen Durchführung einer kalibrierten Bohrwiderstandsmessung zunächst ein erfindungsgemäßer Kalibrieradapter mit einem Prüfkörper vorgegebener Dichte bzw. Festigkeit herangezogen und an dem Bohrwiderstandsmessgerät aufgesetzt und befestigt, so dass der Durchtrittskanal des Kalibrieradapters mit der Bohrnadelführung in der Bohrnadelführungshülse fluchtet. Das Bohrwiderstandsmessgerät wird an dem Messobjekt angesetzt, und die Bohrwiderstandsmessung durch Vorschub der Bohrnadel begonnen. Zunächst durchdringt die Bohrnadel den im Kalibrieradapter angeordneten Prüfkörper und erfasst dabei über den Bohrwiderstand das mit der Dichte bzw. Festigkeit des Prüfkörpers korrelierte Kalibriersignal S1. Ohne das Bohrwiderstandsmessgerät abzusetzen, wird die Messung fortgesetzt und die Bohrnadel weiter vorgeschoben, so dass sie nach Passage des Kalibrieradapters in das Messobjekt eindringt und dabei über den Bohrwiderstand das mit der Dichte bzw, Festigkeit des Messobjekts korrelierte Messsignal S2 erfasst. Aus der Differenz zwischen dem Bohrwiderstand-Kalibriersignal, dem Bohrwiderstand-Messsignal und der vorgegebenen Dichte bzw. Festigkeit des Prüfkörpers kann die Dichte bzw. Festigkeit des Messobjekts berechnet werden.

Ausgabegrößen des Bohrwiderstandsmessgeräts können das Bohrwiderstand-Messsignal S2, das Kalibriersignal S1 oder die Differenz der beiden Signale sein, so dass der Nutzer die Berechnung der gesuchten Größe, sei es die Dichte des Messobjekts oder eine andere mit dem Bohrwiderstand korrelierbare Größe, mit Kenntnis der entsprechenden Größe des Prüfkörpers einfach vornehmen kann.

**Fig. 9** zeigt die Bohrkurve einer kalibrierten Messung, bei der das Messsignal S2 kleiner als das Kalibriersignal S1 ist, was anzeigt, dass das Holz eine geringere Dichte als der Prüfkörper hat. Zur Auswertung der Bohrkurve wird die Differenz zwischen den Messsignalen S1 und S2 als Messwert genutzt, so dass artifizielle

Effekte auf die Messung, wie sie etwa durch die Verwendung einer stumpfen Bohrnadel entstehen können, eliminiert werden. Dies zeigt sich insbesondere beim Vergleich der Bohrkurve aus **Fig. 9** (Bohrnadel in Ordnung) mit der Bohrkurve aus **Fig. 11** (stumpfe Bohrnadel), die eine gleiche Differenz S1-S2 liefern, obwohl sich die absoluten Bohrwiderstände deutlich unterscheiden. Durch die vorangegangene Kalibrierung mittels des Prüfkörpers wird das gleiche Ergebnis erhalten, unabhängig von der Qualität der benutzten Bohrnadel.

**Fig. 10** zeigt ein Beispiel einer Bohrkurve mit dem Kalibriersignal S1 und dem Messsignal S2, aus dem hervorgeht, dass das hölzerne Messobjekt eine Dichte aufweist, die größer ist als die des Prüfmaterials.

Viele Bohrwiderstandsmessgeräte mit integrierter Computereinheit liefern bereits ausgewertete Messkurven, so kann über Menüpunkte, die beispielsweise auf einem Display 13 des Bohrwiderstandsmessgeräts 10 (vgl. **Fig. 2****)** angezeigt werden, ausgewählt werden, ob der Messwert als Bohrwiderstand oder Dichte oder gegebenenfalls auch eine andere Werkstoffgröße ausgegeben werden soll.

Die kalibrierte Messung kann vorteilhaft auch automatisch verarbeitet werden, wenn in den Computer des Bohrwiderstandsmessgeräts eine entsprechende Eingabe zu der interessierenden Werkstoffgröße des Materials, z. B. Dichte oder Festigkeit etc. des Prüfkörpers, gemacht wird. Dann kann die gesuchte Größe exakt und direkt ausgegeben werden; weitere Berechnungen erübrigen sich.

Bei einem Bohrwiderstandsmessgerät mit integriertem Computer ist es ferner denkbar, dass in einem Menü anwählbar und auf einem Display anzeigbar ist, ob eine durchzuführende Messung mit oder ohne Kalibrierung mit der entsprechenden zugehörigen Auswertung erfolgt.

## Patentansprüche

1. Kalibrieradapter (1) für ein Bohrwiderstandsmessgerät (10), das zumindest
- eine mit einem Antrieb gekoppelte Bohrnadelaufnahme,
- eine Bohrnadel (15),
- eine Führungshülse (12) mit einer Bohrnadelaustrittsöffnung und
- eine Vorrichtung zur Bohrwiderstanderfassung und -ausgabe
aufweist,
**dadurch gekennzeichnet, dass**
der Kalibrieradapter (1) einen Grundkörper (3) hat, der
- eine Kopplungsvorrichtung (5) zur lösbaren Befestigung des Kalibrieradapters (1) an dem Bohrwiderstandsmessgerät (10) vor der Bohrnadelaustrittsöffnung, und
- einen Bohrnadel-Durchtrittskanal (33), der sich durch den Grundkörper (3) erstreckt und der derart ausgebildet ist, dass er in einer Befestigungsanordnung des Kalibrieradapters (1) an dem Bohrwiderstandsmessgerät (10) mit der Bohrnadelaustrittsöffnung fluchtet, und
- zumindest einen Aufnahmeraum (31) für zumindest einen Prüfkörper (2) aufweist,
wobei eine durch den Durchtrittskanal (33) bereitgestellte Fluchtachse den zumindest einen Aufnahmeraum (31) schneidet, und wobei der zumindest eine Aufnahmeraum (31) zumindest ein Lagerelement (36, 37) für den Prüfkörper (2) aufweist, das eine Dreh- oder Verschiebbarkeit des Prüfkörpers (2) in einer Ebene orthogonal zur Fluchtachse bereitstellt.

2. Kalibrieradapter (1) nach Anspruch 1,
**dadurch gekennzeichnet, dass**
der Kalibrieradapter (1) einen Deckel (32) aufweist, der den Grundkörper (3) ergänzt und abnehmbar ist und den zumindest einen Aufnahmeraum (31) zusammen mit dem Grundkörper (3) begrenzt, wobei eine im Deckel (32) vorliegende Durchtrittsöffnung (33') mit dem Durchtrittskanal (33) im Grundkörper (3) fluchtet.

3. Kalibrieradapter (1) nach Anspruch 1 oder 2, **dadurch gekennzeichnet, dass**
der Kalibrieradapter (1)
- eine Fixiervorrichtung (38) für den drehbar und/oder verschiebbar gelagerten Prüfkörper (2) aufweist, und/oder
- eine Stellvorrichtung, eine Erfassungsvorrichtung und/oder eine Anzeigevorrichtung zum manuellen und/oder automatischen Positionieren des Prüfkörpers (2) in dem zumindert einen Aufnahmeraum (31) aufweist.

4. Kalibrieradapter (1) nach zumindest einem der Ansprüche 1 bis 3,
**dadurch gekennzeichnet, dass**
die Kopplungsvorrichtung (5) Rast- oder Klemmvorrichtungen, bevorzugt Schrauben, besonders bevorzugt Rändelschrauben (5), zum Festklemmen des Kalibrieradapters (1) an der Bohrnadelführungshülse (12) oder an einer die Bohrnadelführungshülse (12) einfassenden Kappe (14) aufweist.

5. Kalibriervorrichtung für ein Bohrwiderstandsmessgerät (10), das zumindest
- eine mit einem Antrieb gekoppelte Bohrnadelaufnahme,
- eine Bohrnadel (15),
- eine Führungshülse (12) mit einer Bohrnadelaustrittsöffnung und
- eine Vorrichtung zur Bohrwiderstanderfassung und -ausgabe
aufweist,
**dadurch gekennzeichnet, dass**
die Kalibriervorrichtung einen Kalibrieradapter (1) nach zumindest einem der Ansprüche 1 bis 4 und zumindest einen Prüfkörper (2) aufweist, der Abmessungen und eine Form aufweist, die eine Anordnung des Prüfkörpers (2) in dem zumindest einen Aufnahmeraum (31) des Kalibrieradapters (1) gestatten, so dass ein Prüfabschnitt des Prüfkörpers (2) vollständig in der durch den Durchtrittskanal (33) definierten Fluchtachse positionierbar ist.

6. Kalibriervorrichtung nach Anspruch 5, **dadurch gekennzeichnet, dass** die Kalibriervorrichtung zumindest zwei Prüfkörper (2) unterschiedlicher Werkstoffe mit bekannten, mit dem Bohrwiderstand korrelierten Werkstoffgrößen aufweist, wobei jeweils einer der zumindest zwei Prüfkörper (2) in dem zumindest einen Aufnahmeraum (31) angeordnet ist.

7. Kalibriervorrichtung nach Anspruch 5 oder 6, wobei der Prüfkörper (2) eine Prüfscheibe (2) mit einer zentrischen Bohrung (21) ist,
**dadurch gekennzeichnet, dass**
der zumindest eine Aufnahmeraum (31) benachbart zu dem Durchtrittskanal (33) einen parallel zu der Fluchtachse angeordneten Lagerstift (36) zur drehbaren Anordnung der Prüfscheibe (2) aufweist, wobei die in dem Aufnahmeraum (31) aufgenommene Prüfscheibe (2) bevorzugt teilweise aus dem Grundkörper (3) ragt, und besonders bevorzugt einen profilierten Rand aufweist.

8. Kalibriervorrichtung nach Anspruch 5 oder 6, wobei der Prüfkörper (2) eine leistenartige Prüfplatte ist, **dadurch gekennzeichnet, dass**
der zumindest eine Aufnahmeraum (31) sich orthogonal zu der Fluchtachse durch den Grundkörper (3) hindurch erstreckt, so dass die Prüfplatte durch den Aufnahmeraum durchschiebbar ist.

9. Bohrwiderstandsmessvorrichtung, umfassend ein Bohrwiderstandsmessgerät (10) und eine Kalibriervorrichtung, **dadurch gekennzeichnet, dass**
die Kalibriervorrichtung eine Kalibriervorrichtung nach zumindest einem der Ansprüche 5 bis 8 ist und der Kalibrieradapter (1) der Kalibriervorrichtung mittels der Kopplungsvorrichtung (5) des Grundkörpers (3) lösbar an dem Bohrwiderstandsmessgerät (10) vor der Bohrnadelaustrittsöffnung befestigt ist.

10. Verfahren zur kalibrierten Bohrwiderstandsmessung, **umfassend die Schritte**
- Bereitstellen eines Kalibrieradapters (1) nach zumindest einem der Ansprüche 1 bis 4 mit einem Prüfkörper (2) aus einem vorgegebenen Werkstoff mit einer vorgegebenen, mit dem Bohrwiderstand korrelierten Werkstoffgröße,
- Aufsetzen und Befestigen des Kalibrieradapters (1) an dem Bohrwiderstandsmessgerät (10) vor der Bohrnadelaustrittsöffnung, so dass der Durchtrittskanal (33) des Kalibrieradapters (1) mit der Bohrnadelaustrittsöffnung fluchtet,
- Durchführen einer Bohrwiderstandsmessung mit dem Bohrwiderstandsmessgerät (10) an einem Messobjekt (100) unter gleichmäßigem Vorschub der Bohrnadel (15), dabei
- zunächst Durchdringen des in dem Kalibrieradapter (1) vorliegenden Prüfkörpers (2) mit der Bohrnadel (15) und Erfassen eines Bohrwiderstand-Kalibriersignals (S1),
- weiter Vorschieben und Einführen der Bohrnadel (15) in das Messobjekt (100) und Erfassen eines Bohrwiderstand-Messsignals (S2),
- Berechnen der Werkstoffgröße des Messobjekts (100) basierend auf einer Differenz zwischen dem Kalibriersignal (S1) und dem Messsignal (S2) und der vorgegebenen, mit dem Bohrwiderstand korrelierten Werkstoffgröße des Prüfkörpers (2), dann Zurückziehen der Bohrnadel (15) und
- Durchführen einer weiteren Bohrwiderstandsmessung, wobei der Prüfkörper (2) In seiner Stellung verbleibt und bei der weiteren Messung das Bohrwiderstand-Kalibriersignal (S1) der ersten Messung herangezogen wird, und/oder
- Verändern der Lage des Prüfkörpers (2) durch Drehen und/oder Verschieben in dem Aufnahmeraum (31) in einer Ebene orthogonal zu der Fluchtachse, so dass ein nicht durchbohrter Prüfabschnitt des Prüfkörpers (2) in der Fluchtachse zu liegen kommt, und Durchführen einer weiteren kalibrierten Bohrwiderstandsmessung, und/oder
- Austauschen des Prüfkörpers (2).

11. Verfahren nach Anspruch 9 oder 10,
**umfassend den Schritt**
zum Bereitstellen des Kalibrieradapters (1) Auswählen eines Prüfkörpers (2) mit einer Werkstoffgröße, deren Größenordnung im Bereich einer zu erwartenden Werkstoffgröße des Messobjekts (100) liegt.

## Claims

1. Calibration adapter (1) for a drilling resistance measuring device (10) that comprises
- at least one drilling needle receptacle coupled to a drive,
- a drilling needle (15),
- a guide sleeve (12) comprising a drilling needle outlet opening, and
- a device for drilling resistance recording and output,
**characterized in that**
the calibration adapter (1) comprises a main body (3) that comprises
- a coupling device (5) for releasable fastening of the calibration adapter (1) on the drilling resistance measuring device (10) in front of the drilling needle outlet opening, and
- a drilling needle passage (33) which extends through the main body (3) and is designed such that it is aligned with the drilling needle outlet opening in a fastening arrangement of the calibration adapter (1) on the drilling resistance measuring device (10), and
- at least one receiving space (31) for at least one test body (2), wherein an alignment axis provided by the passage (33) intersects the at least one receiving space (31),
and wherein the at least one receiving space (31) comprises at least one bearing element (36,37) for the test body (2) that provides rotatability or slidability of the test body (2) in a plane orthogonal to the alignment axis.

2. Calibration adapter (1) according to claim 1,
**characterized in that**
the calibration adapter (1) comprises a cover (32) that supplements the main body (3) and is removable and delimits the at least one receiving space (31) together with the main body (3), wherein a through opening (33') provided in the cover (32) is aligned with the passage (33) in the main body (3).

3. Calibration adapter (1) according to claim 1 or 2,
**characterized in that** the calibration adapter (1) comprises
- a fixation device (38) for the rotatably and/or slidably supported test body (2), and/or
- an actuating device, a detection device, and/or a display device for manual and/or automatic positioning of the test body (2) in the at least one receiving space (31).

4. Calibration adapter (1) according to at least one of the claims 1 to 3,
**characterized in that**
the coupling device (5) comprises locking or clamping devices, preferably screws, particularly preferred knurled screws (5), for fastening by clamping the calibration adapter (1) on the drilling needle guide sleeve (12) or on a cap (14) surrounding the drilling needle guide sleeve (12).

5. Calibration apparatus for a drilling resistance measuring device (10) that comprises at least
- a drilling needle receptacle coupled with a drive,
- a drilling needle (15),
- a guide sleeve (12) with a drilling needle outlet opening, and
- a device for drilling resistance recording and output,
**characterized in that**
the calibration apparatus comprises a calibration adapter (1) according to at least one of the claims 1 to 5 and at least one test body (2) having dimensions and a shape that enable an arrangement of the test body (2) in the at least one receiving space (31) of the calibration adapter (1) so that a test section of the test body (2) is positionable completely on the alignment axis defined by the passage (33).

6. Calibration apparatus according to claim 5, **characterized in that** the calibration apparatus comprises at least two test bodies (2) of different materials with known material parameters correlated with the drilling resistance wherein one of the at least two test bodies (2) is arranged in the at least one receiving space (31), respectively.

7. Calibration apparatus according to claim 5 or 6, wherein the test body (2) is a test disk (2) with a central bore (21),
**characterized in that**
the at least one receiving space (31) comprises adjacent to the passage (33) a bearing pin (36) arranged parallel to the alignment axis for rotatable arrangement of the test disk (2), wherein the test disk (2) received in the receiving space (31) preferably projects partially out of the main body (3) and particularly preferred has a profiled rim.

8. Calibration apparatus according to claim 6 or 7, wherein the test body (2) is a strip-like test plate, **characterized in that**
the at least one receiving space (31) extends orthogonally to the alignment axis through the main body (3) so that the test plate can be pushed through the receiving space

9. Drilling resistance measuring apparatus comprising a drilling resistance measuring device (10) and a calibration apparatus,
**characterized in that**
the calibration apparatus is a calibration apparatus according to at least one of the claims 5 to 8 and the calibration adapter (1) of the calibration apparatus is detachably fastened by means of the coupling device (5) of the main body (3) on the drilling resistance measuring device (10) in front of the drilling needle outlet opening.

10. Method for calibrated drilling resistance measurement, **comprising the steps of**
- providing a calibration adapter (1) according to at least one of the claims 1 to 4 with a test body (2) of a predetermined material with a predetermined material parameter correlated with the drilling resistance,
- attaching and fastening the calibration adapter (1) to the drilling resistance measuring device (10) in front of the drilling needle outlet opening so that the passage (33) of the calibration adapter (1) is aligned with the drilling needle outlet opening,
- performing a drilling resistance measurement with the drilling resistance measuring device (10) on an object to be measured (100) with uniform feed of the drilling needle (15), in this context
- first penetrating the test body (2) present within the calibration adapter (1) by the drilling needle (15) and recording a drilling resistance calibration signal (S1),
- further advancing and inserting the drilling needle (15) into the object to be measured (100) and recording a drilling resistance measurement signal (S2),
- calculating the parameter of the object to be measured (100) based on a difference between the calibration signal (S1) and the measurement signal (S2) and the predetermined material parameter correlated with the drilling resistance of the test body (2), then retracting the drilling needle (15) and
- performing a further drilling resistance measurement, wherein the test body (2) remains in its position and in the further measurement the drilling resistance calibration signal (S1) of the first measurement is utilized, and/or
- changing the position of the test body (2) by rotation and/or sliding in the receiving space (31) in a plane orthogonal to the alignment axis so that a test section that has not yet been drilled through of the test body (2) is positioned on the alignment axis and performing a further calibrated drilling resistance measurement, and/or
- exchanging the test body (2).

11. Method according to claim 9 or 10,
comprising the step of,
for providing the calibration adapter (1), selecting a test body (2) with a material parameter whose magnitude is in the range of an expected material parameter of the object to be measured (100).

## Revendications

1. Adaptateur d'étalonnage (1) pour un appareil de mesure de la résistance de perçage (10) qui présente au moins
- une réception d'aiguille de perçage couplée à un entraînement,
- une aiguille de perçage (15),
- un manchon de guidage (12) comprenant une ouverture de sortie d'aiguille de perçage et
- un dispositif pour détecter la résistance et la distribution de perçage,
**caractérisé en ce que**
l'adaptateur d'étalonnage (1) a un corps de base (3), qui présente
- un dispositif de couplage (5) pour la fixation séparable de l'adaptateur d'étalonnage (1) à l'appareil de mesure de la résistance de perçage (10) devant l'ouverture de sortie d'aiguille de perçage, et
- un canal de passage d'aiguille de perçage (33) qui s'étend au travers du corps de base (3) et qui est ainsi conçu qu'il affleure avec l'ouverture de sortie d'aiguille de perçage dans un agencement de fixation de l'adaptateur d'étalonnage (1) sur l'appareil de mesure de la résistance de perçage (10), et
- au moins un espace de réception (31) pour au moins un corps d'essai (2),
dans lequel un axe d'affleurement fourni par le canal de passage (33) coupe l'au moins un espace de réception (31) et dans lequel l'au moins un espace de réception (31) présente au moins un élément de palier pour le corps d'essai (2) qui fournit une possibilité de rotation ou de déplacement du corps d'essai (2) dans un plan orthogonal à l'axe d'affleurement.

2. Adaptateur d'étalonnage (1) selon la revendication 1, **caractérisé en ce que** l'adaptateur d'étalonnage (1) présente un couvercle (32) qui complète le corps de base (3) et est amovible et délimite l'au moins un espace de réception (31) conjointement avec le corps de base (3), dans lequel une ouverture de passage présente dans le couvercle (32) affleure avec le canal de passage (33) dans le corps de base (3).

3. Adaptateur d'étalonnage (1) selon la revendication 1 ou 2, **caractérisé en ce que**
l'adaptateur d'étalonnage (1) présente
- un dispositif de fixation (38) pour le corps d'essai (2) disposé en pouvant être tourné et/ou déplacé, et/ou
- un dispositif de réglage, un dispositif de détection et/ou un dispositif d'affichage pour le positionnement manuel et/ou automatique du corps d'essai (2) dans l'au moins un espace de réception (31).

4. Adaptateur d'étalonnage (1) selon au moins l'une des revendications 1 à 3,
**caractérisé en ce que**
le dispositif de couplage (5) présente des dispositifs d'encliquetage ou de serrage, de préférence des vis, particulièrement de préférence des vis moletées (5) pour serrer l'adaptateur d'étalonnage (1) sur le manchon de guidage d'aiguille de perçage (12) ou sur un bouchon (14) enchâssant le manchon de guidage d'aiguille de perçage (12).

5. Dispositif d'étalonnage pour un appareil de mesure de la résistance de perçage (10) qui présente au moins
- une réception d'aiguille de perçage couplée à un entraînement,
- une aiguille de perçage (15),
- un manchon de guidage (12) comprenant une ouverture de sortie d'aiguille de perçage et
- un dispositif pour détecter la résistance et la distribution de perçage,
**caractérisé en ce que**
le dispositif d'étalonnage présente un adaptateur d'étalonnage (1) selon au moins une des revendications 1 à 4 et au moins un corps d'essai (2) qui présente des mesurages et une forme qui permet un agencement du corps d'essai (2) dans l'au moins un espace de réception (31) de l'adaptateur d'étalonnage (1) de sorte qu'un tronçon d'essai du corps d'essai (2) peut être positionné intégralement dans l'axe d'affleurement défini par le canal de passage (33).

6. Dispositif d'étalonnage selon la revendication 5, **caractérisé en ce que** le dispositif d'étalonnage présente au moins deux corps d'essai (2) de matériaux différents avec des grandeurs de matériau connues, corrélées à la résistance de perçage, dans lequel respectivement un des au moins deux corps d'essai (2) est disposé dans l'au moins un espace de réception (31).

7. Dispositif d'étalonnage selon la revendication 5 ou 6, dans lequel le corps d'essai (2) est un disque d'essai (2) avec un perçage central (21),
**caractérisé en ce que**
l'au moins un espace de réception (31) présente dans le voisinage du canal de passage (33), un ergot de commande (36) disposé parallèle à l'axe d'affleurement, pour l'agencement du disque d'essai (2) avec une possibilité de rotation, dans lequel le disque d'essai (2) reçu dans l'espace de réception (31) fait saillie de préférence partiellement hors du corps de base (3) et présente particulièrement de préférence un bord profilé.

8. Dispositif d'étalonnage selon la revendication 5 ou 6, dans lequel le corps d'essai (2) est un plateau d'essai en forme de baguette, **caractérisé en ce que** l'au moins un espace de réception (31) s'étend au travers du corps de base (3) de façon orthogonale à l'axe d'affleurement, de telle sorte que le plateau d'essai peut être poussé au travers de l'espace de réception.

9. Dispositif de mesure de la résistance de perçage, comprenant un appareil de mesure de la résistance de perçage (10) et un dispositif d'étalonnage,
**caractérisé en ce que**
le dispositif d'étalonnage est un dispositif d'étalonnage selon au moins l'une des revendications 5 à 8 et l'adaptateur d'étalonnage (1) du dispositif d'étalonnage est fixé de façon séparable sur l'appareil de mesure de la résistance de perçage (10) devant l'ouverture de sortie d'aiguille de perçage, au moyen du dispositif de couplage (5) du corps de base (3).

10. Procédé de mesure étalonnée de la résistance de perçage, comprenant **les étapes suivantes :**
- fourniture d'un adaptateur d'étalonnage (1) selon au moins l'une des revendications 1 à 4 comprenant un corps d'essai (2) dans un matériau prédéfini avec une grandeur de matériau prédéfinie, corrélée avec la résistance de perçage,
- placement et fixation de l'adaptateur d'étalonnage (1) sur l'appareil de mesure de la résistance de perçage (10) devant l'ouverture de sortie d'aiguille de perçage, de façon à ce que le canal de passage (33) de l'adaptateur d'étalonnage (1) affleure avec l'ouverture de sortie d'aiguille de perçage,
- exécution d'une mesure de résistance de perçage avec l'appareil de mesure de la résistance de perçage (10) sur un objet de mesure (100) par une avancée régulière de l'aiguille de perçage (15), avec
- d'abord la pénétration du corps d'essai (2) présent dans l'adaptateur d'étalonnage (1) avec l'aiguille de perçage (15) et détection d'un signal d'étalonnage de résistance de perçage (S1),
- puis la poussée et l'introduction de l'aiguille de perçage (15) dans l'objet de mesure (100) et détection d'un signal de mesure de résistance de perçage (S2),
- calcul de la grandeur de matériau de l'objet de mesure (100) en se basant sur une différente entre le signal d'étalonnage (S1) et le signal de mesure (S2) et la grandeur de matériau prédéfinie, corrélée avec la résistance de perçage du corps d'essai (2), puis retrait de l'aiguille de perçage (15) et
- exécution d'une mesure supplémentaire de la résistance de perçage, dans lequel le corps d'essai (2) reste dans sa position et lors de la mesure supplémentaire, le signal d'étalonnage de résistance de perçage (S1) est rapproché de la première mesure et/ou
- modification de la position du corps d'essai (2) par rotation et/ou déplacement dans l'espace de réception (31) dans un plan orthogonal à l'axe d'affleurement, de façon à ce qu'un tronçon d'essai non percé du corps d'essai (2) vienne se placer dans l'axe d'affleurement et exécution d'une mesure de résistance de perçage étalonnée supplémentaire, et/ou
- remplacement du corps d'essai (2).

11. Procédé selon la revendication 9 ou 10, comprenant l'étape, pour fournir l'adaptateur d'étalonnage (1), de sélection d'un corps d'essai (2) avec une grandeur de matériau dont l'ordre de grandeur est situé dans la plage d'une grandeur de matériau attendue de l'objet de mesure (100).
